(19) Europäisches Patentamt
European Patent Office
Office européen des brevets

(11) Veröffentlichungsnummer : **0 082 385**
**B1**

(12) **EUROPÄISCHE PATENTSCHRIFT**

(45) Veröffentlichungstag der Patentschrift : **30.07.86**

(51) Int. Cl.$^4$ : **A 61 K 31/505**

(21) Anmeldenummer : **82111233.1**

(22) Anmeldetag : **04.12.82**

(54) **1-(3-Nitrophenyl)pyrido(2.3-d)pyrimidin-2.4(1H, 3H)-dione und 1-(3-Nitrophenyl)chinazolin-2.4(1H, 3H)-dione als cutane Arzneimittel.**

(30) Priorität : **18.12.81 DE 3150271**

(43) Veröffentlichungstag der Anmeldung : **29.06.83 Patentblatt 83/26**

(45) Bekanntmachung des Hinweises auf die Patenterteilung : **30.07.86 Patentblatt 86/31**

(84) Benannte Vertragsstaaten : **AT BE CH DE FR GB IT LI LU NL SE**

(56) Entgegenhaltungen :
**EP-A- 0 040 793**
**DE-A- 2 459 060**
**DE-A- 2 459 090**

(73) Patentinhaber : **Troponwerke GmbH & Co. KG**
**Berliner Strasse 156**
**D-5000 Köln 80 (DE)**

(72) Erfinder : **Pelster, Bernhard, Dr.**
**An den Weiden 7a**
**D-5201 St. Augustin 1 (DE)**
Erfinder : **Horstmann, Harald, Dr.**
**Claudiusweg 19**
**D-5600 Wuppertal 1 (DE)**

(74) Vertreter : **Jesse, Ralf-Rüdiger, Dr. et al**
**Bayer AG Konzernverwaltung RP Patentabteilung**
**D-5090 Leverkusen 1 Bayerwerk (DE)**

**EP 0 082 385 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft cutan applizierbare Arzneimittel, die Verbindungen der allgemeinen Formel

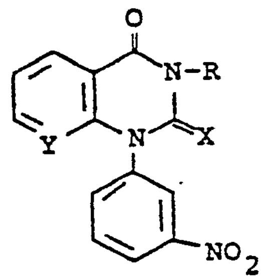

(I)

in welcher

R für Wasserstoff, gegebenenfalls ungesättigtes niederes Alkyl oder Aralkyl mit jeweils 1 bis 20 Kohlenstoffatomen,

X für Sauerstoff oder Schwefel und

Y für Stickstoff oder eine CH-Gruppe steht,

enthalten und die in Form von Suppositorien, Suspensionen, Emulsionen, Sprays, Puder, Gelen, Salben, Pasten, Cremes und Lösungen, gegebenenfalls in mikroverkapselter Form, jedoch ausgenommen Tragacanth-Salzlösungen, vorliegen.

Die erfindungsgemäßen Arzneimittel dienen insbesondere der Behandlung entzündlicher und/oder schmerzhafter Prozesse.

Niederes Alkyl bzw. Aralkyl bedeutet Reste mit 1 bis 20 C-Atomen, vorzugsweise 1 bis 6 C-Atomen, besonders bevorzugt 1 bis 4 C-Atomen.

Die pharmazeutische Verwendung von Verbindungen der allgemeinen Formel I und ihre Herstellung ist bereits in der DE-OS 2 459 060 und DE-OS 2 459 090 beschrieben. Pharmakologische Wirkungen wurden nachgewiesen, indem man die Substanzen z. B. in Tragacanth-Salzlösungen Nagern i. p. oder p. o. applizierte. Orale Applikation der Substanzen ist in vielen Fällen mit erheblichen Nebenwirkungen wie Nausea oder Tachyphylaxie verbunden.

Überraschenderweise wurde nun gefunden, daß Verbindungen der allgemeinen Formel I in geeigneter Form bei topischer Anwendung stark entzündungshemmend wirken, ohne die oben beschriebenen Nebenwirkungen auszulösen.

Zur vorliegenden Erfindung gehören ferner Verfahren zur Herstellung der erfindungsgemäßen Arzneimittel und die Verwendung von Verbindungen der allgemeinen Formel I zur Herstellung von cutan applizierbaren Arzneimitteln.

Unter cutaner Anwendung im Sinne der vorliegenden Erfindung wird die topische Anwendung allgemein sowie die Anwendung bzw. Aufbringung auf Schleimhäute ausgenommen der Mundschleimhaut verstanden.

Die erfindungsgemäßen Arzneimittel enthalten den oder die Wirkstoffe neben den üblichen Trägerstoffen, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltigemittel, z. B. Glycerin, (d) Lösungsverzögerer, z. B. Paraffin und (e) Resorptionsbeschleuniger, z. B. quarternäre Ammonium-verbindungen, (f) Netzmittel, z. B. Cetylalkohol, Glycerinmonostearat, (g) Adsorptionsmittel, z. B. Kaolin und Bentonit und (h) Gleitmittel, z. B. Talkum, Calcium- und Magnesiumstearat und feste Polyethylenglykole oder Gemische der unter (a) bis (h) aufgeführten Stoffe.

Die Arzneimittel können auch so zusammengesetzt sein, daß sie den oder Wirkstoffe nur verzögert abgeben, wobei als Einbettungsmassen z. B. Polymersubstanzen und Wachse verwendet werden können.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z. B. Wasser, Ethylenalkohol, Isopropylalkohol, Ethylcarbonat, Ethylacetat, Benzylalkohol, Benzylbenzoat, Propylenglykol, 1,3-Butylenglykol, Öle, insbesondere Baumwollsaatöl, Maiskeimöl, Olivenöl und Sesamöl, Glycerin, Glycerinformal, Tetrahydrofurfurylalkohol, Polyethylenglykole und Fettsäureester des Sorbitans oder Gemische dieser Stoffe enthalten.

Für die Herstellung von Lösungen bzw. Suspensionen oder Emulsionen der cutan zu applizierenden Substanzen werden erfindungsgemäß besonders bevorzugt Dimethylsulfoxid, Dimethylformamid, Dimethylacetamid, Lutrol, Cremophor, Ricinusöl, Erdnußöl, N-Methylpyrrolidon, Etofenanamat und Salicylsäuremethylester eingesetzt.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte

Isostearylalkohole, Polyoxyethylensorbit- und Sorbitanester, mikrokristalline Cellulose, Aluminiummetahydroxid, Bentonit, Agar-Agar oder Gemische dieser Stoffe enthalten.

Die genannten Formulierungsformen können auch gefärbte und farbgebende Mittel, Konservierungsstoffe sowie geruchsverbessernde Zusätze, z. B. Pfefferminzöl und Eukalyptusöl enthalten.

Die therapeutisch wirksamen Verbindungen sollen in den erfindungsgemäßen Arzneimitteln vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die erfindungsgemäßen Arzneimittel, sind in der Human- und Veterinärmedizin bei der Bekämpfung von entzündlichen und/oder schmerzhaften Prozessen verwendbar.

Im allgemeinen hat es sich sowohl in der Human- als auch in der Veterinärmedizin als vorteilhaft erwiesen, die erfindungsgemäßen Arzneimittel so zu dosieren, daß Wirkstoff-Gesamtmengen von etwa 0,1 bis etwa 100, vorzugsweise 0,5 bis 10 mg/kg Körpergewicht je 24 Stunden, gegebenenfalls mit Hilfe mehrerer Einzelgaben erreicht werden. Eine Einzelgabe enthält den oder die Wirkstoffe vorzugsweise in Mengen von etwa 0,1 bis etwa 70, insbesondere 0,1 bis 2,0 mg/kg Körpergewicht. Es kann jedoch erforderlich sein, von den genannten Dosierungen abzuweichen und zwar in Abhängigkeit von der Art des zu behandelnden Objekts und der Art und der Schwere der Erkrankung.

Topisch anwendbar sind insbesondere die 1-(3-Nitrophenyl)pyrido[2.3-d]pyrimidin-2.4(1H, 3H)-dione der allgemeinen Formel I, worin Y für N steht, und die 1-(3-Nitrophenyl)chinazolin-2.4-(1H, 3H)-dione, worin Y für CH steht. R hat in beiden bevorzugten Fällen die Bedeutung von Wasserstoff, niedrigeren Alkylgruppen mit bevorzugt 1 bis 6 Kohlenstoffatomen, niedrigeren Alkenylgruppen mit bevorzugt 3 bis 5 Kohlenstoffatomen, Halogenalkyl, Propargyl, Cyclopropylmethyl, niedrigeren Halogenalkylgruppen mit bevorzugt 1 bis 3 Kohlenstoffatomen, niedrigeren Trihalogenalkylgruppen mit bevorzugt 1 bis 3 Kohlenstoffatomen, Acetoxyethyl, niedrigeren Hydroxyalkylgruppen, bevorzugt mit 2 bis 3 Kohlenstoffatomen, niedrigeren Alkoxyalkylgruppen mit bevorzugt 2 bis 4 Kohlenstoffatomen, Vinyloxyethyl, Hydroxyethoxyethyl, Carboxymethyl, Ethoxycarbonylmethyl, 2,3-Epoxypropyl, Diethylaminoethyl, 4-Methylpiperazinethyl, Benzyl, Phenethyl oder Cinnamyl und X die Bedeutung von Sauerstoff oder Schwefel.

Besonders bevorzugte Substanzen sind :

Verbindung 1 1-(3-Nitrophenyl)-3-ethylchinazolin-2.4(1H, 3H)-dion

Verbindung 2 1-(3-Nitrophenyl)-3-(2-fluorethyl)-chinazolin-2,4(1H, 3H)dion

Verbindung 3 1-(3-Nitrophenyl)-3-ethylpyrido [2.3-d] pyrimidin-2,4(1H, 3H)dion

Die ödemhemmende Wirkung der Testsubstanzen nach topischer Anwendung wurde mit Hilfe des durch Kaolin induzierten Ödems der Rattenpfote ermittelt.

Die Versuche wurden mit männlichen Ratten (Stamm : Bor : WISW (SPF-Cpb ; Gewicht : 150-220 g) durchgeführt. Eine Stunde vor Auslösung des Ödems (0,1 ml Kaolinsuspension/Tier subplantar) wurde den Tieren die im jeweiligen Lösungsmittel gelöste Substanz auf die am Tage zuvor geschorene Rückenhaut aufgetragen und leicht ein-massiert ; zur Kontrolle wurde eine zweite Tiergruppe mit reinem Lösungsmittel ohne Substanz behandelt. Die Pfotenvolumina wurden nach der Methode von F. Kemper und G. Ameln, Z. ges. exp. Med. *131*, 407 (1959), gemessen, wobei die Differenz von Pfotenvolumen 5 Stunden nach der Ödemprovokation und dem normalen Pfotenvolumen das Ödemvolumen ergibt.

Zunächst wurde beispielhaft die $DE_{50}$ für Verb. 1 nach cutaner Applikation in Dimethylsulfoxid bestimmt. Es wurde gefunden, daß durch cutane Applikation von 0,3 mg/kg der Verb. 1 in Dimethylsulfoxid derselbe antiphlogistische Effekt erzielt werden kann, wie nach oraler Applikation von 0,3 mg/kg.

In weiterführenden Untersuchungen wurden gleiche Mengen der Verb. 1 (jeweils 0,3 mg/kg) in verschiedenen Lösungsmitteln cutan appliziert. Danach sind Dimethylsulfoxid, Dimethylformamid, Dimethylacetamid, Salicylsäuremethylester und bevorzugt N-Methylpyrrolidon im Hinblick auf die antiphlogistische Wirkung besonders als Lösungsmittel für die cutane Applikation von Verb. 1 geeignet. (Tab. 1)

(Siehe Tabelle 1 Seite 4 f.)

Tabelle 1

Antiphlogistische Wirkung von Verbindung 1

0,3 mg/kg, Applikationsart : cutan, Ratte ♀, n = 5 (n = Anzahl der Ratten)

| Lösungsmittel | % Hemmung des Kaolinödems |
|---|---|
| Dimethylsulfoxid | 48.3 |
| Dimethylformamid | 35.0 |
| Dimethylacetamid | 50.5 |
| Lutrol | 18.3 |
| Cremophor | 14.2 |
| Ricinusöl | 14.2 |
| Erdnußöl | 16.7 |
| N-Methylpyrrolidon | 63.2 |
| Salicylsäuremethylester | 77.1 |
| (Salicyclsäuremethylester allein | 39.4) |

In gleicher Weise sind auch die anderen Verbindungen der allgemeinen Formel I cutan anwendbar (Tab. 2).

Tabelle 2

Hemmung des Kaolinödems nach cutaner Applikation von Lösungen der Testverbindungen in Dimethylsulfoxid

| Verbindung | mg/kg (cutan) | % Hemmung des Kaolinödems | $DE_{50}$ (mg/kg) Kaolinödem p.o. |
|---|---|---|---|
| 1 | 0.3 | 48.3 | 0.3 |
| 2 | 0.3 | 56.0 | 0.2 |
| 3 | 0.3 | 79.8 | 0.1 |

Der erfindungsgemäße Vorteil der cutanen gegenüber der oralen Anwendung besteht darin, daß durch die Wahl dieser speziellen Applikationsart pharmakologisch wertvolle Substanzwirkungen erhalben bleiben, unerwünschte Nebenwirkungen jedoch vermieden werden können, d. h., durch cutane Anwendung in geeigneten Lösungsmitteln werden die Verbindungen gemäß der allgemeinen Formel I überhaupt erst pharmazeutisch nutzbar.

Die Vermeidung der Nebenwirkungen durch cutane Applikation von Verb. 1 ist nachfolgend beispielhaft für Verb. 1 beschrieben.

1. Sowohl nach oraler als auch intramuskulärer Applikation von Verb. 1 treten bereits ab einer Dosis von etwa 0.1 mg/kg beim Hund starke emetische Erscheinungen auf. Diese emetische Wirkung beim Hund bleibt auch nach cutaner Applikation von 5 mg/kg der Substanz aus.

2. Verb. 1 induziert bei Ratten nach oralen Gaben über mehrere Tage nach bereits drei bis vier Tagen eine deutliche Tachyphylaxie. Dieser Wirkungsverlust der untersuchten Substanz wird durch ihre cutane Applikation vollständig vermieden. Verb. 1 zeigt auch nach 6 Tagen anhaltender topischer Vorbehandlung der Ratten mit in Dimethylsulfoxid gelöster Substanz (0.3 mg/kg/Tag) seine eindeutige ödemhemmende Wirkung.

Es war daher überraschend, daß die Verbindungen der allgemeinen Formel I in geeigneter Form bei topischer Anwendung stark entzündungshemmend wirken, ohne die vorstehend beschriebenen Nebenwirkungen auszulösen.

Im folgenden werden noch einige typische erfindungsgemäße Mischungen, die sich besonders gut für die cutane Applikation eignen, angegeben. Die Mischungen werden wie vorstehend beschrieben zubereitet.

TVX bedeutet 1-(3-nitrophenyl)-3-ethylchinazolin-2,4-(1H, 3H)-dion ; Texapon®, Natriumlaurylsulfat ; IPM Isopropylmyristat ; und Aerosil® Siliziumdioxid.

| | |
|---|---|
| TVX | 1.0 |
| Texapon® | 40.0 |
| Isopropylmyristat | 180.0 |
| Vaseline®, weiß | 739.0 |
| Paraffin | 40.0 |
| | 1000.0 |

| | |
|---|---|
| TVX | 1.0 |
| Texapon® | 40.0 |
| Isopropylmyristat | 180.0 |
| Vaseline®, weiß | 734.0 |
| Paraffin | 20.0 |
| Aerosil® | 25.0 |
| | 1 000.0 |

| | |
|---|---|
| Polyäthylenglykol 300 | 500.0 |
| Polyäthylenglykol 1 500 | 499.0 |
| TVX | 1.0 |
| | 1 000.0 |

| | |
|---|---|
| Cetylstearylalkohol | 300.0 |
| Paraffin, dickflüssig | 348.0 |
| Vaseline®, weiß | 350.0 |
| TVX | 2.0 |
| | 1 000.0 |

| | |
|---|---|
| TVX | 0.1 |
| Milchsäureethylester | 9.9 |
| | 10.0 |

| | |
|---|---|
| TVX | 0.1 |
| Aceton | 6.9 |
| IPM | 3.0 |
| | 10.0 |

| | |
|---|---|
| TVX | 0.1 |
| Essigsäureethylester | 6.9 |
| IPM | 3.0 |
| | 10.0 |

Aufbereitung von TVX - Hemmung des Carrageenan-Pfotenödems der Ratte

| | | % Hemmung |
|---|---|---|
| TVX | 0.03 | |
| Milchsäureethylester | 9.97 | |
| | 10.00 | 42.0 |
| TVX | 0.03 | |
| Milchsäureethylester | 6.97 | |
| IPM | 3.00 | |
| | 10.00 | 50.6 |

(Fortsetzung)

| | | % Hemmung |
|---|---|---|
| TVX | 0.03 | |
| Essigsäureethylester | 9.97 | |
| | 10.00 | 31.0 |
| TVX | 0.03 | |
| Essigsäureethylester | 6.97 | |
| IPM | 3.00 | |
| | 10.00 | 64.1 |
| TVX | 0.03 | |
| Aceton | 9.97 | |
| | 10.00 | 41.7 |
| TVX | 0.03 | |
| Aceton | 6.97 | |
| IPM | 3.00 | |
| | 10.00 | 51.3 |
| TVX | 0.03 | |
| IPM | 3.00 | |
| Tween® | 2.00 | |
| Isopropanol | 4.97 | |
| | 10.00 | 55.1 |
| TVX | 0.03 | |
| Tween® | 0.50 | |
| Dowanol® DPM | 9.47 | |
| | 10.00 | 62.7 |

**Patentansprüche**

1. Cutan applizierbares Arzneimittel, enthaltend eine Verbindung der allgemeinen Formel

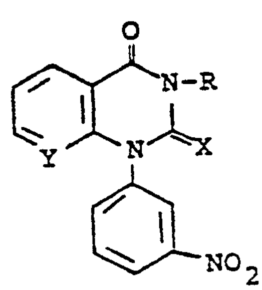

(I)

in welcher

R Wasserstoff, gegebenenfalls ungesättigtes niederes Alkyl oder Aralkyl mit jeweils 1 bis 20 Kohlenstoffatomen,

X Sauerstoff oder Schwefel und

Y Stickstoff oder CH bedeutet,

in Form von Suppositorien, Suspensionen, Emulsionen, Sprays, Puder, Gelen, Salben, Pasten, Cremes und Lösungen, gegebenenfalls in mikroverkapselter Form, jedoch ausgenommen Tragacanth-Salzlösungen.

2. Cutan applizierbares Arzneimittel gemäß Anspruch 1 enthaltend 0,1 bis 99,5 Gew.-% einer oder mehrerer Verbindungen der allgemeinen Formel I gemäß Anspruch 1.

3. Verfahren zur Herstellung von Arzneimitteln, dadurch gekennzeichnet, daß eine Verbindung der allgemeinen Formel I gemäß Anspruch 1 in eine cutane Applikationsform überführt wird.

4. Verwendung von Verbindungen der allgemeinen Formel I gemäß Anspruch 1 zur Herstellung von cutan applizierbaren Arzneimitteln.

**Claims**

1. Cutaneously administrable medicament containing a compound of the general formula

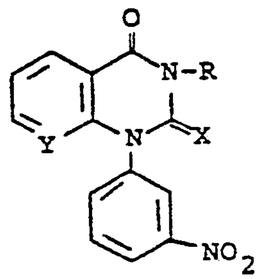

(I)

in which

R denotes hydrogen, optionally unsaturated lower alkyl or aralkyl with in each case 1 to 20 carbon atoms,

X denotes oxygen or sulphur and

Y denotes nitrogen or CH,

in the form of suppositories, suspensions, emulsions, sprays, powders, gels, ointments, pastes, creams and solutions, optionally in a microencapsulated form, but with the exception of tragacanth salt solutions.

2. Cutaneously administrable medicament according to Claim 1, containing 0.1 to 99.5 % by weight of one or more compounds of the general formula I according to Claim 1.

3. Process for the preparation of medicaments, characterised in that a compound of the formula I according to Claim 1 is converted into a cutaneous form of administration.

4. Use of compounds of the general formula I according to Claim 1 for the preparation of cutaneously administrable medicaments.

**Revendications**

1. Médicament applicable par voie cutanée, contenant un composé de formule générale

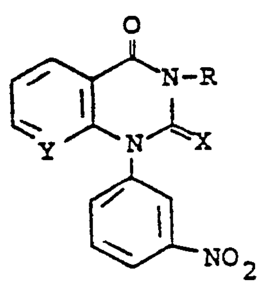

(I)

dans laquelle

R représente l'hydrogène, un groupe alkyle inférieur éventuellement insaturé ou un groupe aralkyle ayant 1 à 20 atomes de carbone,

X représente l'oxygène ou le soufre et

Y représente l'azote ou le groupe CH,

sous la forme de suppositoires, de suspensions, d'émulsions, de compositions pulvérisables, de poudres, de gels, de pommades, de pâtes, de crèmes et de solutions, le cas échéant sous la forme micro-encapsulée, mais à l'exception de solutions salines de gomme adragante.

2. Médicament applicable par voie cutanée suivant la revendication 1, contenant 0,1 à 99,5 % en poids d'un ou plusieurs composés de formule générale I suivant la revendication 1.

3. Procédé de préparation de médicaments, caractérisé en ce qu'un composé de formule générale I suivant la revendication 1 est mis sous une forme d'application cutanée.

4. Utilisation de composés de formule générale I suivant la revendication 1 pour la préparation de médicaments applicables par voie cutanée.